# EUROPEAN PATENT SPECIFICATION

(11) **EP 0 410 326 B1**
(45) Date of publication and mention of the grant of the patent: **21.10.1998**
(21) Application number: 90113985.7
(22) Date of filing: 20.07.1990
(51) Int. Cl.: C12P 19/06, C08B 37/00

(54) **Genetic control of acetylation and pyruvylation of xanthan gum**
Genetische Kontrolle zur Acetylierung und Pyruvylierung von Xanthangummi
Contrôle génétique pour l'acétylation et la pyruvylation de gomme de xanthane

(30) Priority: 25.07.1989 US 384621
(43) Date of publication of application: 30.01.1991
(73) Proprietor: MONSANTO COMPANY, St. Louis, Missouri 63167 (US)
(72) Inventor: Doherty, Daniel H., Boulder, CO 80303 (US); Hassler, Randal A., Lafayette, CO 80026 (US)
(74) Representative: Grünecker, Kinkeldey, Stockmair & Schwanhäusser Anwaltssozietät

(56) References cited:
- WO-A-87/05939
- MACROMOLECULES, vol. 16, no. 5, May 1983, Easton, PA (US); M. RINAUDO et al., pp. 816-819/

## Description

### BACKGROUND OF THE INVENTION

This invention relates to polysaccharide polymers and to a process for preparing same. In particular, it relates to xanthan-based polysaccharide polymers, defined herein as polymers structurally similar to xanthan gum and produced by components of the xanthan biosynthetic pathway, including those xanthan-based polymers modified so that the outer mannose can be specifically acetylated but not pyruvylated, pyruvylated but not acetylated, or unmodified while the inner mannose can be independently controlled to be acetylated or unmodified.

Xanthan gum is produced by bacteria of the genus Xanthomonas, in particular by microorganisms of the species X. campestris. Xanthan gum is a widely used product due to its unusual physical properties, i.e., its extremely high specific viscosity and its pseudoplasticity. It is commonly used in foods as a thickening agent and in secondary or tertiary oil recovery as a mobility control and profile modification agent, as well a in petroleum drilling fluids.

Chemically, xanthan gum is an anionic heteropolysaccharide. The repeating unit of the polymer is a pentamer composed of five sugar moieties, specifically two glucose, one glucuronic acid and two mannose moieties. These sugar residues are arranged such that the glucose moieties form the backbone of the polymer chain, with side chains of mannose-glucuronic acid-mannose residues generally extending from alternate glucose moieties. Usually, this basic structure is specifically acetylated and pyruvylated, as described, for example, by Janson, P.E., Kenne, L., and Lindberg, B., in Carbohydrate Research, 45:275-282 (1975) and Melton, L.D., Minot, L., Rees, D.A., and Sanderson, G.R., in Carbohydrate Research, 46:245-257 (1976), each of which is specifically incorporated herein by reference. The extent of acetylation and pyruvylation is known to vary. The structure of xanthan gum is depicted in formula I below:

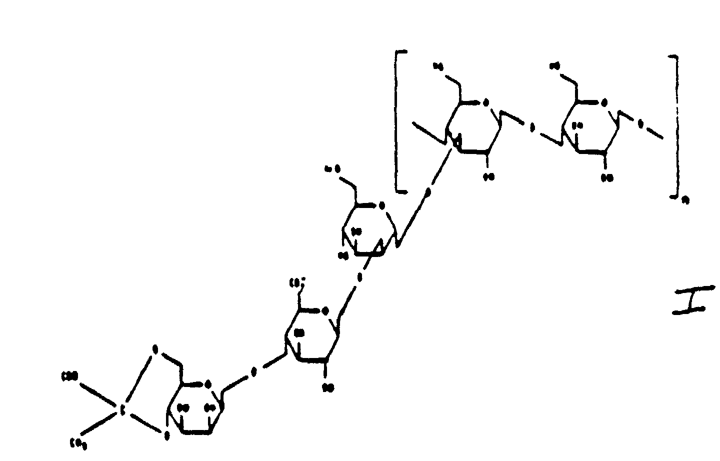

In spite of the broad utility of naturally-occurring xanthan gum, there are some situations where its physical properties become limiting. In particular, in secondary or tertiary oil recovery it is not uncommon for the temperature of the oil bearing reservoir and the salt concentrations in the reservoir brine to be higher than are optimal for xanthan solutions. When these conditions occur, xanthan can precipitate, flocculate and/or lose its viscosity. Therefore, new viscosifying products which perform well at various conditions encountered during oil recovery, such as high temperature and high salt concentrations would be desirable.

The present invention discloses a family of xanthan-based polysaccharides having improved properties relative to naturally-occurring xanthan gum. Modifications of xanthan gum have been previously described. For example, Bradshaw et al. (Carbohydrate Polymers, 3:23-38 (1983)) describe methods for preparing chemically-modified xanthan gum which is deacetylated or depyruvylated. Various means of chemically deacetylating xanthan gum produced by Xanthomonas campestris also are described in U.S. Patent Nos. 3,000,790 and 3,054,689. To date, the predominant method utilized for these deacetylation processes has been chemical removal of the acetate moieties from normally acetylated xanthan gum. It has been found that chemical processes for deacetylating xanthan gums result in a number of undesirable side effects and may cause hydrolysis of the glycosidic backbone, resulting in an irreversible change in the conformation of the molecule and lowered molecular weight.

Xanthan gum can be chemically depyruvylated as well, as described by Holzwarth and Ogletree in Carbo. Res. 76:277-280 (1979). This chemical method of depyruvylation also can alter the xanthan polymeric unit and/or cause hydrolysis of the glycosidic backbone. While a strain of X. campestris has been described in U.S. Patent No. 4,296,203 which produces non-pyruvylated xanthan gum, this non-pyruvylated gum was either fully acetylated or deacetylated using chemical means. According to Rinaudo et al., Macromolecules 16, pp. 816-819, 1983, the acetate as well as the pyruvate content of Xanthan gums vary due to culture conditions and also due to post fermentation processing. In Carbohydrate Research 206, pp.87-103, 1990, Xanthan gums having more than 8.1 % pyruvate are disclosed.

In view of the aforementioned disadvantages of chemical deacetylation and chemical depyruvulation, there is a demand for Xanthomonas strains producing naturally not occurring Xanthan gums. W087/05939 discloses the selection of Xanthomonas campestris mutant strains obtained by random mutagenesis.

It is an object of the present invention to provide a process for preparing polysaccharide xanthan polymers in which the inner mannose is acetylated or unmodified while the outer mannose is acetylated, pyruvylated or unmodified, avoiding the necessity of chemical modification.

It is also an object of the present invention to provide an in vitro method for obtaining microorganisms having the ability to produce members of this family of polysaccharide polymers in vivo.

Additional objects and advantages of the invention will be set forth in part in the description which follows, and in part will be obvious form the description, or may be learned by practice of the invention. The objects and advantages may be realized and attained by means of the instrumentalities and combinations particularly pointed out in the appended claims.

### SUMMARY OF THE INVENTION

To achieve the objects and in accordance with the purposes of the invention, as embodied and broadly described herein, there is provided a process for preparing a process for preparing a water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, said process comprising:
(a) obtaining a Xanthomonas which is incapable of expressing a functional gene F protein of the xanthan gum gene cluster;
(b) culturing said Xanthomonas under conditions sufficient to produce said polysaccharide polymer.

Further, there is provided a process for preparing a water-soluble polysaccharide polymer comprising repeating pentamer units having a D-glucose: D-mannose: D-glucuronic acid ratio of about 2:2:1, wherein D-glucose moieties are linked in a beta[1,4] configuration, inner D-mannose moieties are linked in an alpha-[1,3] configuration primarily to alternate glucose moieties, the D-glucuronic acid moieties are linked to a beta[1,2] configuration to said inner mannose moieties, and outer mannose moieties are linked to said glucuronic acid moieties in a beta-[1,4] configuration, wherein said outer mannose moieties are not acetylated and a portion of said inner mannose moieties are acetylated, said process comprising:
(a) obtaining a Xanthomonas which is incapable of expressing a functional gene G protein of the xanthan gum gene cluster;
(b) culturing said Xanthomonas under conditions sufficient to produce said polysaccharide polymer.

The polysaccharide polymers of this invention can be made generally by genetic manipulations of the microbial biosynthetic pathways which lead to the production of polysaccharides. In particular, microbial pathways for the production of xanthan gum may be manipulated to create an in vivo or in vitro system for the production of an altered polymeric unit. Thus, systems can be created through the use of mutated Acetylase I, Acetylase II and Ketalase genes, in particular, to create polysaccharides which are acetylated or pyruvylated to varying degrees.

To further achieve the objects and in accordance with the purposes of the present invention, there is provided a xanthan gum wherein the inner mannose moieties are acetylated at the 6-0 position. Another structure contemplates both the inner and outer mannose moieties being acetylated. A further structure is provided having a portion of the outer mannose moieties pyruvylated at the 4-6 position and a portion acetylated. Another structure is provided with the outer mannose moieties pyruvylated at the 4-6 position and the inner mannose moieties acetylated at the 6-0 position. Two additional structures are provided, one having the outer mannose moieties pyruvylated at the 4-6 position and the other having the outer mannose moieties acetylated.

It is understood that both the foregoing general description and the following detailed description are exemplary and explanatory only and are not restrictive of the invention, as claimed. The accompanying drawings, which are incorporated in and constitute a part of this specification, illustrate various embodiments of the invention and, together with the description, serve to explain the principles of the invention.

### BRIEF DESCRIPTION OF THE DRAWINGS

Figure 1a shows the BamHI restriction map of a 16 kb region of the chromosome of X. campestris that contains a cluster of twelve genes required for biosynthesis of xanthan and also shows the approximate locations of these twelve genes relative to the BamHI restriction map.

Figure 1b shows some restriction sites in and around genes F and G and the DNA sequence at the junction of genes F and G.

Figure 2 depicts the construction of a deletion mutation (delCla) within gene F of the gum gene cluster.

Figure 3a shows the structure of plasmid p13delCla derived from pRK290-H336 by in vivo insertion of transposon TnK12 into the vector portion of pRK290-H336 in the approximate location shown in the figure and the subsequent deletion as described in Figure 2 of the 660 base pair ClaI DNA fragment within gene F.

Figure 3b shows the structure of plasmid pH336KBmldelCla which is similar in structure to p13delCla, containing the same 660 base pair deletion within gene F. This plasmid contains an insertion mutation (KBm1) at the BamHI site within gene G. The DNA fragment inserted there is a BamHI restriction fragment carrying the kanamycin-resistance gene of plasmid pUC4-K.

Figure 4 depicts the chemical structure, and a schematic representation, of the repeating unit of the polytetramer variant of xanthan gum.

Figure 5a shows the structure of plasmid pHA3KBm2delCla derived from pRK290-HA3 which is identical to pRK290-H336 except that it does not contain the 1.4 kb and 1.5 kb BamHI fragments of the X. campestris gum biosynthetic operon DNA and therefore lacks genes B and C but contains genes D through M. pHA3KBm2delCla contains the gene F deletion mutation described in Figure 2 and an insertion mutation in the BamHI site of gene I. The inserted DNA is again a BamHI restriction fragment of pCU4-K which carries a gene conferring kanamycin resistance.

Figure 5b shows the extent of a chromosomal deletion mutation present in X. campestris strain X1106 with genes D through M being deleted, while B and C are intact and functional in the chromosome.

Figure 6 depicts schematic representations of the structures of repeating units of the polysaccharides produced by wild-type X. campestris and mutants defective in genes F, G, or L and all possible combinations of mutations in genes F, G, and L. Abbreviations used are: G = glucose; M = mannose; GA = glucuronic acid; Ac = acetate; Pyr = pyruvate.

### DETAILED DESCRIPTION OF THE INVENTION

Reference will now be made in detail to the presently preferred embodiments of the present invention which, together with the following examples, serve to explain the principles of the invention.

The polysaccharide polymers of the present invention have been described in detail above. These polysaccharide polymers can be produced in vitro with a cell-free enzyme system or can be produced in vivo by growing cells of an appropriate mutant strain. Other means of preparing the polysaccharide polymers are also described below.

### In Vitro Polysaccharide Synthesis

The basic method relating to the use of a cell-free system to make non-variant xanthan gum is described by Ielpi, L., Couso, R.O., and Dankert, M.A. in FEBS Letters 130: 253-256 (1981), specifically incorporated herein by reference. It has been found that a modified version of this method may be employed to create the variant polysaccharides of this invention.

For this novel, modified method, the in vitro cell-free system is prepared generally by lysing cells of a microorganism of the genus Xanthomonas, preferably Xanthomonas campestris, in the presence of a suitable buffer, preferably including EDTA, and obtaining the appropriate biosynthetic enzymes which are able to subsequently process exogenously added substrates. This general method for this in vitro system in described in U.S. Patent No. 4,713,449 of Vanderslice et al., specifically incorporated herein by reference.Alternate means of lysis may be used, including but not limited to sonication, French Pressure cell, detergent treatment, enzyme treatment and combinations thereof.

Generally, to produce the variant polysaccharides of the present invention, a lysate of a microorganism possessing the enzymes required to assemble the desired polysaccharide is incubated with the appropriate substrates, which, depending on the gum desired, may include UDP-glucose, GDP-mannose, UDP-glucuronic acid, acetyl-CoA and phosphoenolpyruvate. The choice of substrates is dependent on the polysaccharide which it is desired to produce. For example, a non-acetylated polysaccharide is obtained by eliminating acetyl-CoA as a substrate. Similarly, a non-pyruvylated gum is obtained by elminating phosphoenol-pyruvate as a substrate. Chemical and/or enzymatic treatment of the cell lysates in order to deplete endogeneous substrates will be evident to one skilled in the art.

In addition, cell-free systems may be created from mutant organisms deficient in one or more of the enzymes of the xanthan biosynthetic pathway. Such mutant-derived cell lysates would produce the variant gums described herein, either due solely to the mutation or due to the mutation in combination with a withheld substrate.

The biosynthetic process may, in one embodiment, be monitored by the incorporation of radiolabeled substrates into the polymeric units. Other methods may also be used to allow identification of the biosynthetic intermediates that are known to those of ordinary skill in the art. In particular, chromatographic methods have been developed to separate and to identify the oligosaccharide intermediates. These include thin layer chromatography and high-performance liquid chromatography.

The cell-free biosynthesis of xanthan has been found to be a time-dependent, sequential process that is dependent on the addition of all three specific nucleotides. The background of non-specific incorporation of labeled substrate is minimal and does not interfere with the detection of the xanthan-specific polymer in the gum fraction.

The involvement of lipid carriers, specifically isoprenoid pyrophosphate, has been shown in several polysaccharide biosynthetic pathways. Additionally, the involvement of pyrophosphoryl-linked lipid carrier in xanthan biosynthesis has been demonstrated. Thus, the xanthan biosynthetic intermediates have been found to be recoverable in the organic soluble fraction with these carrier lipids. The recovered oligosaccharide can subsequently be freed from the carrier lipid by mild acid hydrolysis, for example, pH 2 for 20 minutes at 90°C and dephosphorylated with alkaline phosphatase for analysis.

Using these methods for recovery of intermediate products, it has been discovered that, under in vitro conditions, certain lysates of X. campestris mutants will produce non-acetylated or non-pyruvylated xanthan gum even in the presence of all substrates required for non-variant gum synthesis. In light of the teachings herein, these methods will enable one skilled in the art to identify cell lysates which produce other altered polysaccharides.

### In Vivo Polysaccharide Synthesis

The development of the cell-free synthesis process for the polysaccharides described above demonstrated that various Xanthomonas campestris cells have all the enzymes necessary to synthesize xanthan-based polymers that have the mannose residues acetylated, pyruvylated or unmodified.

Furthermore, for whole cells to synthesize non-acetylated xanthan gum, a means of blocking the acetylation of either the inner or outer mannose during xanthan gum synthesis would be required. Additionally, for the whole cells to synthesize non-acetylated, non-pyruvylated xanthan gum, a means of blocking xanthan gum synthesis at both the acetylation and pyruvylation steps would be required. In one embodiment of the present invention, mutagenesis was employed to alter some of the genes responsible for these various reactions.

Transposons, including but not limited to Tn10, TnK12 (Tn10 del16del17KanR), and Tn903, can be used to mutagenize Xanthomonas campestris. These transposons, in one embodiment, confer resistance to tetracycline or kanamycin. Transposons have the ability to insert themselves into genes wherein they cause mutations by interrupting the coding sequence. The transposons can be introduced into Xanthamonas campestis on various vectors, including on so-called suicide vectors, such as pRK2013. Vector pRK2013, as described by Ditta, G., Corbin, D. and Helinski, D.R. in Proc. Natl. Acad. Sci. U.S.A., 77:7347-7351 (1980), specifically incorporated herein by reference, has the ability to transfer itself into non-enteric bacteria, such as Xanthomonas campestris, but cannot replicate in that host. Thus, if the suicide vector is introduced into a population of Xanthomonas campestris cells and that population is subsequently challenged with either tetracycline or kanamycin, the individuals which survive are those in which one of the transposons has inserted into the genome of Xanthonomas campestris. Survivors of such a challenge can be screened for those which have lost the ability to make xanthan gum. Such mutants may appear less mucoid than mold-type Xanthonomas campestris.

Other means of mutagenesis can be employed to generate mutants that do not acetylate and/or pyruvylate the gums they produce. Such means will readily occur to one skilled in the art, and include, without limitation, irradiation, recombinant DNA technology (in particular, as described in United States Patent Application Serial No. 333,868 of Capage et al., entitled "Recombinant-DNA Mediated Production of Xanthan Gum," filed April 3, 1989, and incorporated specifically herein by reference, and in Example 1 below) and chemical mutagen treatment. Examples of such mutagenesis procedures have been described by Miller, J.H. in Experiments in Molecular Genetics (1972); Davis, R.W., Bostein, D. and Roth, J.R. in Advanced Bacterial Genetics (1980); and Maniatis, T., Fritsch, E.F. and Sambrook, J. in Molecular Cloning (1982), Cold Spring Harbor.

Alternatively, appropriate mutants can be detected by assaying the culture broth of each mutant for the presence of the desired polysaccharide, e.g., xanthan gum which has the outer mannose acetylated but not pyruvylated, pyruvylated but not acetylated, both pyruvylated and acetylated or unmodified while the inner mannose is acetylated or unmodified. Thus, mutants can be found which appear to be blocked at various positions of the xanthan gum pathway. Mutants of Xanthomonas campestris which produce xanthan gum that is acetylated at the inner mannose and acetylated at the outer mannose (X1397), acetylated at the inner mannose and pyruvylated at the outer mannose (X1398), acetylated at the inner mannose and unmodified at the outer mannose (X1399), unmodified at the inner mannose with a portion of the outer mannose moieties pyruvylated and a portion acetylated (X1400), unmodified at the inner mannose and acetylated at the outer mannose (X1401), and unmodified at the inner mannose and pyruvylated at the outer mannose (X1402), and unmodified at the inner mannose and unmodified at the outer mannose (X1403), have each been placed on deposit at the American Type Culture Collection, Rockville, Maryland, under Accession Nos. 68033, 68034, 68035, 68036, 68037, 68038 and 68039, respectively.

It is not beyond the scope of the invention to employ enzyme inhibitors of Acetylase I, Acetylase II and Ketalase to arrive at the same products. Still other alternatives for producing this family of polysaccharides are contemplated, including enzymatic and chemical degradation of natural xanthan gum.

The mutants can be grown under conditions generally known in the art for growth of wild-type Xanthomonas campestris. For example, they can be grown on suitable assimilable carbon sources such as glucose, sucrose, maltose, starch, complex carbohydrates such as molasses or corn syrup, various organic acids and the like. Mixtures of carbon sources can also be employed. The concentration of carbon source supplied is often between 10 and 60 grams per liter. Also necessary for growth are an assimilable source of organic or inorganic nitrogen, generally between about 0.1 and 10.0 grams per liter, and minerals, the choice of which are easily within the skill of the art. Examples of suitable nitrogen sources are ammonium salts, nitrate, urea, yeast extract, peptone, or other hydrolyzed proteinaceous materials or mixtures thereof. Examples of suitable minerals include phosphorus, sulfur, potassium, sodium, iron, magnesium; these are often added with a chelating agent such as EDTA or citric acid.

Optimal temperatures for growth of Xanthomonas campestris generally are between 18 and 35°C, preferably between about 27° and 30°C. Xanthomonas campestris cells are grown aerobically by supplying air or oxygen so that an adequate level of dissolved oxygen is maintained, for example, above about 10% of saturation. Preferably, the level is kept above about 20%. The pH often is maintained at about 6.0 to 8.0, preferably at about 6.5 to 7.5.

The polysaccharides of the present invention can be recovered from fermentation broths by a suitable means. Precipitation with isopropanol, ethanol, or other suitable alcohol readily yields the polysaccharides of this invention. Generally, alcohols are added to a concentration of about 50 to 75%, on the basis of volume, preferably in the presence of potassium chloride, sodium chloride or other salt. Alterntively, the polymers can be recovered from the broth by ultra-filtration.

Mobility control solutions for use in enhanced oil recovery may also be prepared from the variant polysaccharide polymers disclosed herein. Solutions of the polysaccharide polymers at concentrations of from about 50 to about 3000 ppm are appropriate for such mobility control solutions. Other known additives may also be used in combination with these solutions to further enhance oil recovery. Such additives include, for example, surfactants, alkaline agents or metal or organic crosslinking agents.

The polysaccharide polymers, like xanthan gum, can also be used as thickening agents in foods, cosmetics, medicinal formulations, paper sizing, drilling muds, printing inks, and the like and as a gelling agent. In addition, they can be used to reduce frictional drag of fluid flow in pipes.

### EXAMPLES

The following examples illustrate certain of the preferred embodiments of the present invention.

### EXAMPLE 1

This example demonstrates that there are two X. campestris genes which encode enzymes that catalyze acetylation of xanthan gum.

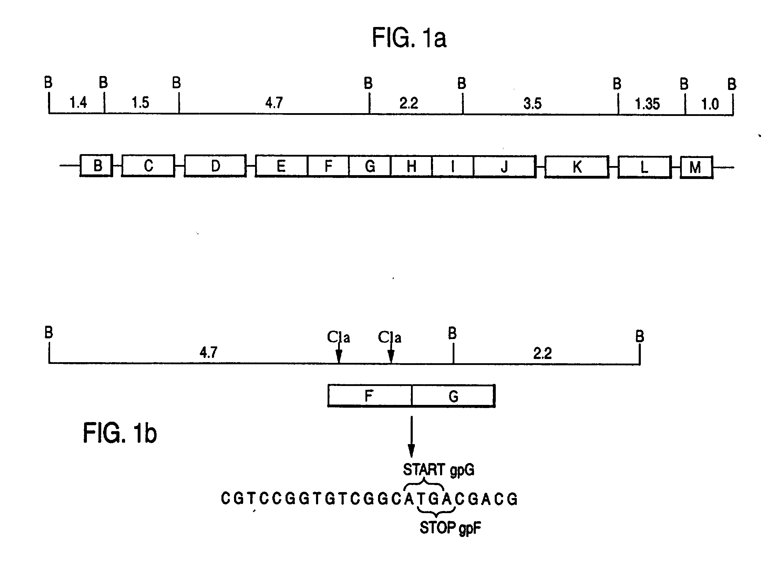

Capage et al., EP-A-0 326 544 described the nucleotide sequence of a 16 kb segment of X. campestris DNA that contains a gene cluster required for xanthan gum biosynthesis. Mutations were isolated that inactivated each of the genes identified by the DNA sequence. The phenotypes of mutant strains carrying these mutations were determined. Mutations in gene F (see Figure 1a), caused by transposon insertion, resulted in production of xanthan gum that contained no detectable acetate. Insertion mutations in gene G did not result in any obvious defect in xanthan gum biosynthesis. Mutants with gene G defects produced high levels of xanthan gum, and this gum contained all of the normal constituents of xanthan in approximately normal molar ratios. On the basis of these initial results, it was concluded that gene F encoded an enzyme that catalyzed the known acetylation of the inner mannose of xanthan, while the activity of the gene G protein remained unknown.

However, when the DNA sequence was used to predict the amino-acid sequences of the products of genes F and G (gpF and gpG), these proteins were found to have extensive homology to one another. This finding indicated that the functions of gpF and gpG might be similar. The phenotypes of mutants defective in gene G were subsequently reexamined, and the compositions of xanthans produced by these mutants were precisely quantitated. These data showed a small (5%-10%) but significant decrease in the acetate content of gum produced by G⁻mutants as compared to wild-type X. campestris. Therefore further experiments were performed to determine what role gpG might have in acetylation of xanthan.

The hypothesis that gpG normally directs 10% of the acetylation of xanthan gum was seemingly contradicted by the observation that transposon insertion mutations in gene F resulted in elimination of acetylation. Clearly, these mutant gums did not retain 10% of the normal acetate content. However, it was possible that insertions in gene F reduced or eliminated the expression of gene G as a result of so-called "polar" effects. Insertions of Tn10 generally reduce expression of genes located downstream, in terms of transcription, from the insertion site as reported by Kleckner, N. et al., in J. Mol. Bio. 97:561-575 (1975). Moreover, the reduction can be quite severe in instances where the downstream gene is "translationally coupled" to the gene containing the insertion mutation as reported by Oppenheim, D.S. and Yanofsky, C., in Genet. 95:785-795 (1980). Translational coupling is a phenomenon wherein the translational stop signal of one gene overlaps the translational start signal of an adjacent downstream gene. In some cases where such coupling occurs, the initiation of translation of the downstream gene is largely or entirely dependent on termination of translation of the upstream gene occurring at the coupler. Thus, insertions in the upstream member of the coupled genes can dramatically reduce, or even eliminate, expression of the downstream gene because these inserts invariably cause frame shifting and premature termination of translation of the upstream gene.

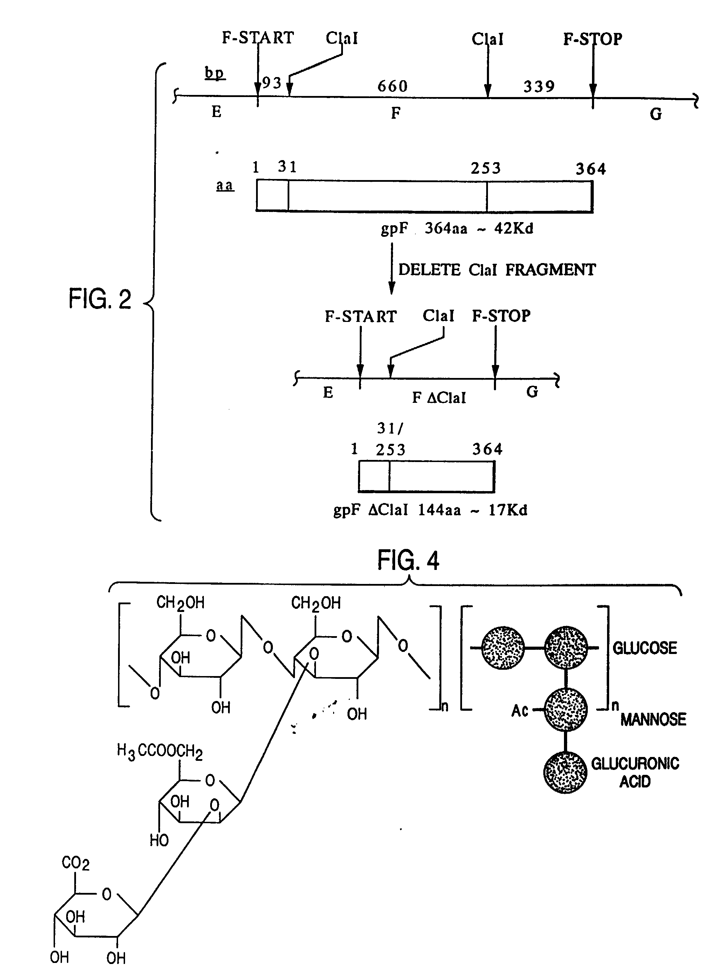

The DNA sequence of the gum gene cluster revealed that the translational stop of gene F does overlap the translational start of gene G, i.e., the two are "coupled" (see Figure 1b). Moreover, the sequence of the translational initiation signal for gene G is not particularly strong, which suggests that the translational coupling might play a significant role in gene G expression. To test this hypothesis, a deletion mutation (as shown in Fig. 2) was constructed within the coding sequence of gene F. This deletion eliminated 660 base pairs between the ClaI sites within gene F. The deleted DNA falls entirely within the coding sequence of gene F, and no foreign DNA is inserted. Thus, the deletion removes a large portion (approximately 60%) of the gene but does not alter the reading frame since the number of base pairs deleted is evenly divisible by 3. The mutant gpF produced by this deletion mutation (gpFdel) is missing 220 amino acids out of a total of 364, but the translational start of gene F and the gene F translational stop, coupled to the start of gene G, remain unaltered. The elimination of two-thirds of the amino acid residues of gpF is very likely, although not certain, to result in elimination of all protein activity. Thus, any residual acetylase activity from this mutant is most apt to be due to activity of gpG.

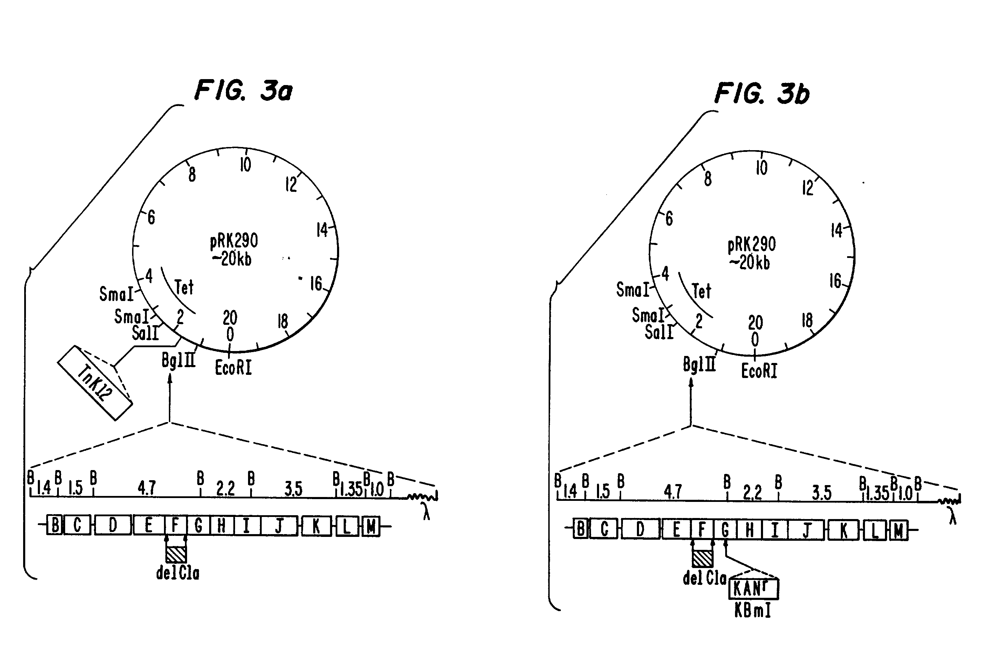

This ClaI deletion mutation was constructed on plasmid pRK290-H336.13 (Figure 3a) which carries an otherwise wild-type gum gene cluster and an insertion of transposon Tn10 del16 del17 KanR described by Way et al., in Gene 32:369-379 (1984) and here termed TnK12, located within the vector position of the plasmid. The TnK12 insertion provides convenient drug resistances for selection of plasmid transfer. The deleted plasmid, termed p13delCla, was transferred into the X. campestris Gum⁻ deletion strain X1231, which is missing genes B - M, and polysaccharide produced by the resulting strain X1231(p13delCla) was analyzed. This gum contained a low but significant amount of acetate; roughly (10-15)% the amount normally found in wild-type xanthan. This result indicated that both gpF and gpG are acetylases and that the bulk of acetylation of xanthan is catalyzed by gpF with a minor component of xanthan acetylation being catalyzed by gpG. However, it remained a possibility that the low level acetylation observed in the mutant X1231 (p13delCla) resulted not from the activity of gpG, but from a residual activity of gpFdel. To address this issue, a double mutant derivative of plasmid pRK290-H336 was constructed. As shown in Figure 3b, this double mutant combined the gene F ClaI deletion mutation and an insertion mutation (KBm1) in gene G. The double mutant plasmid pH336KBm1delCla was transferred into strain X1231, and the polysaccharide produced was analyzed. If the low level acetylation observed in gum produced by X1231(p13delCla) results from the activity of gpG, then the double mutant X1231(pH336KBmldelCla) should eliminate gpG activity by virtue of the insertional mutation in gene G, and no acetylation should be observed. If, however, the real source of acetylating activity in X1231(p13delCla) is the mutant gpFdel, the addition of the gene G insertion should not affect acetylation, and the same 10% level observed in X1231(p13delCla) should be seen in gum produced by the double mutant strain. The polysaccharide produced by strain x1231(pH336KBmldelCla) was found to contain no acetate. This proved that gpG does catalyze acetylation of xanthan and that, in wild-type strains, gpG is responsible for roughly 10% of the total acetylation that is observed.

### EXAMPLE 2

This example demonstrates that the target residue for acetylation by gpG (but not gpF) is the outer mannose of the xanthan repeating unit and that this acetylation is enhanced when pyruvylation of the outer mannose is blocked.

Mutations in gene L (Figure 1a) of the xanthan biosynthetic gene cluster were previously shown to inactivate the ketalase enzyme which catalyzes pyruvylation of the outer mannose. Mutants lacking gpL activity produce xanthan gum devoid of pyruvate. However, initial studies of such mutants revealed that these non-pyruvylated polymers contained unusually high levels of acetate, generally ^f 0.8 acetate/mannose. Thus, the outer mannose can be efficiently acetylated when pyruvylation is genetically blocked and further studies have shown that this acetylation is catalyzed by gpG and not gpF.

In order to examine the interaction of the two acetylase genes with the ketalase gene and each other, a set of eight mutant strains comprising all combinations of mutations in gene F (Acetylase I), gene G (Acetylase II), and gene L (Ketalase) were constructed. The various combinations of mutations were constructed on plasmid pRK290-H336 which contains the entire gum gene cluster.

The gene F mutation employed in these constructions is the in-frame deletion within this gene. As described above, this deletion eliminates 660 base pairs between the ClaI sites located within gene F. The deleted DNA falls entirely within the coding sequence of gene F, and no foreign DNA is inserted. Thus, the deletion removes a large portion (approximately 66%) of the gene but does not alter the reading frame since the number of base pairs deleted is evenly divisible by 3. The mutant gpF produced by this deletion mutation (gpFdel) is missing 220 amino acids out of a total of 364, but the translational start of F and its translational stop coupled to the start of G remain unaltered. The elimination of two-thirds of the amino acid residues of gpF was shown above to eliminate gpF activity.

The gene G mutation used in these mutants is an insertion (KBm1) within gene G at a BamHI site that interrupts the coding sequence of gene G. The inserted DNA is a restriction fragment containing the 1.3 kb Kan^{r} DNA segment of plasmid pUC4-K as described by Vieira, J. and Messing, J., in Gene 19:259-268 (1982), which is ultimately derived from the kanamycin resistance gene of transposon Tn903.

The gene L mutations used were of two types. One is an insertion of transposon TnK12 within the coding region of gene L. The second type is derived from this insertion by deletion of a 3 kb HindIII fragment of TnK12 which carries the genes encoding resistance to kanamycin and streptomycin. In this TnK12 deletion mutation, an insert of 1 kb of TnK12 DNA still remains within the gene L coding sequence and this results in insertional inactivation of the gene L product.

The various combinations of these mutations were constructed on plasmid pRK290-H336 using in vitro recombinant DNA technology. The eight mutant plasmids obtained were then conjugally transferred from E. coli into X. campestris strain X1231 which contains the deletion mutation that eliminates the entire gum gene cluster from the chromosome. The 8 resulting strains X1396-X1403 (Table 1) were then analyzed for polymer production.

**Table 1**

| | Genotype | | |
|---|---|---|---|
| Strain | Acetylase I | Acetylase II | Ketalase |
| X1396^{a} | + | + | + |
| X1397 | + | + | _^{b} |
| X1398 | + | _^{c} | + |
| X1399 | + | _^{c} | _^{d} |
| X1400 | _^{e} | + | + |
| X1401 | _^{e} | + | _^{b} |
| X1402 | _^{e} | _^{c} | + |
| X1403 | _^{e} | _^{c} | _^{d} |

| | | | |
|---|---|---|---|
| ^{a} wild-type, carries TnK12 insertion within pRK290 portion of the plasmid | | | |
| ^{b} TnK12 insertion mutation | | | |
| ^{c} Kan^{r} fragment insertion mutation | | | |
| ^{d} TnK12 deletion derivative insertion mutation | | | |
| ^{e} in-frame, non-polar deletion mutation | | | |

All strains were grown in 50 ml each FXC-RAH-1 medium at pH 7.0 that contained:
3.2 g/l N-Z-amine AS
1.7 g/l MgSo₄.7H₂O
0.7 g/l KH₂PO₄
40 g/l glucose
19.5 g/l (2-(N-morpholino) ethane sulfonic acid)
5-10 mg/l kanamycin
1 mg/l Tetracycline (where applicable)
in 300 ml baffled shake flasks. Temperature was maintained at 30°C. After approximately 60 hours of incubation, the culture broths were diluted with two to four volumes of distilled H₂O and the cells removed by centrifugation at 14,000 - 18,000 x g for 30 minutes at 10°C. Gums were precipitated from the supernatants by the addition of 2-3 volumes of 2-propanol and collected by centrifugation using the conditions described previously. The precipitates were then rehydrated in 100-300 ml of 20 mM NaCl and the precipitations repeated. The gums were finally rehydrated in 100 ml distilled H₂O each. Samples of each were subsequently dialyzed against 4 1 of distilled H₂O for four days with daily H₂O changes in 12,000 - 14,000 MW cutoff cellulose tubing.

Triplicate samples of each purified gum were then concentrated 3-4-fold by vacuum drying and hydrolyzed in 2 M trifluoroacetic acid at 120'C for 2-1/2 hours. After neutralization with 1.2 M Na₂CO₃, the hydrolysates were filtered through 0.45 mim filters and ready for analysis by high-performance liquid chromatography (HPLC).

The analyses were performed using a Beckman HPLC equipped with an Aminex HPX-87H ion exclusion column (300 x 7.8 mm). Organic acids were detected by ultraviolet absorbance at 214 nm. Refractive index was used to detect neutral sugars. The column was run isocratically with 0.01 N H₂SO₄ as the mobile phase at a flow rate of 0.6 ml/minute at 45°C.

The molar ratios of the components in each hydrolysate were calculated using a series of calibration curves based on peak areas for each sugar and organic acid.

The molar ratios of acetate and pyruvate to mannose are shown in Table 2.

**Table 2**

| Molar Ratios of Acetate and Pyruvate to Mannose | | | | | |
|---|---|---|---|---|---|
| Strain | Acetylase I | Acetylase II | Ketalase | Acetate/Mannose | Pyruvate Mannose |
| X1396 | + | + | + | 0.66 | 0.43 |
| X1397 | + | + | - | 1.01 | 0.00 |
| X1398 | + | - | + | 0.63 | 0.36 |
| X1399 | + | - | - | 0.51 | 0.00 |
| X1400 | - | + | + | 0.10 | 0.39 |
| X1401 | - | + | - | 0.47 | 0.00 |
| X1402 | - | - | + | 0.00 | 0.37 |
| X1403 | - | - | - | 0.00 | 0.00 |

The following key observations about the data presented in Table 2 can be made.
1. The 660 bp deletion in gene F inactivated the gene F protein (Acetylase I). See X1402 vs X1398.
2. The gene G protein (Acetylase II) acetylated xanthan and at a much reduced level compared to wild-type when Ketalase was active. See X1400 vs X1396, described in Ex. 4.
3. If Ketalase was inactivated, acetylation by Acetylase II increased dramatically (X1400 vs X1401), described in Ex. 4.
4. The extent of acetylation by Acetylase I did not increase in response to the inactivation of Ketalase. See X1398 vs X1399, described in Ex. 4.
5. Pyruvylation did not vary significantly regardless of the extent of acetylation. See X1396, X1398, X1400, and X1402, described in Ex. 4.

These data indicate that the gene G protein (Acetylase II) catalyzes the acetylation of the external mannose of xanthan. This appears to occur to a limited extent when Ketalase is active, but increases dramatically in Ketalase⁻ mutants. These data indicate that pyruvylation blocks acetylation, but the converse is not true since pyruvylation didn't change significantly regardless of the level of acetylation. The gene F protein (Acetylase I) catalyzes the acetylation of the internal mannose only, and previous data for polytrimer and polytetramer variants of xanthan U.S. Patent No. 4,713,449 and U.S. Application Serial No. 844,335 have shown that Ketalase catalyzes the pyruvylation of the external mannose only.

### EXAMPLE 3

This example demonstrates that gpG (Acetylase II) does not catalyze acetylation of the inner mannose of the xanthan repeating unit.

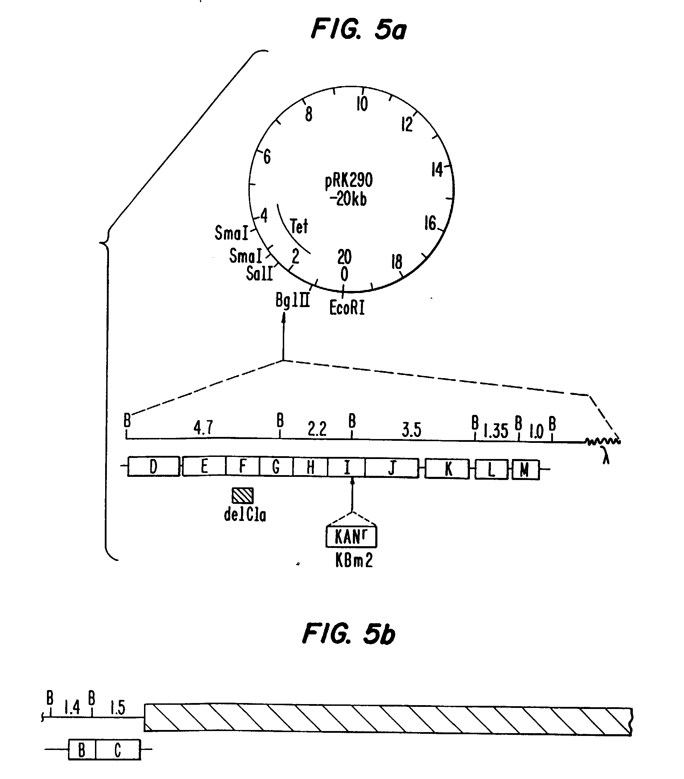

Gene I of the gum gene cluster encodes Transferase V (Figure 1), the enzyme that adds mannose to the lipid-linked tetrasaccharide intermediate in xanthan biosynthesis. This system is described in U.S. Patent No. 4,713,449. Mutations that inactivate gene I lead to the synthesis of a lipid-linked tetrasaccharide. This tetrasaccharide repeating unit is polymerized to yield polytetramer gum, which contains the internal mannose in its normal linkages but lacks the outer mannose normally found on xanthan gum (Figure 4). A double mutant plasmid, pKBm2delCla, was constructed which contains an insertion mutation within gene I and the ClaI deletion mutation within gene F (see Figure 5). The double mutant plasmid pKBm2delCla was transferred into the X. campestris deletion strain X1106 which contains only gum genes B and C in its chromosome. Genes B and C are provided by the chromosome since the mutant plasmid, derived from pRK290-HA3, does not carry B or C but contains all the remaining gum genes, D through M. The resulting strain, X1106(pKBm2delCla) or X1419, was analyzed for polymer composition twice. Both analyses failed to detect acetate in the polymer. This result shows that Acetylase II cannot acetylate the internal mannose of the polytetramer to any significant degree. In this mutant strain, Acetylase II is active because gene G is not mutated and the gene F mutation is the non-polar ClaI deletion which has been shown above not to affect the expression of gene G.

### EXAMPLE 4

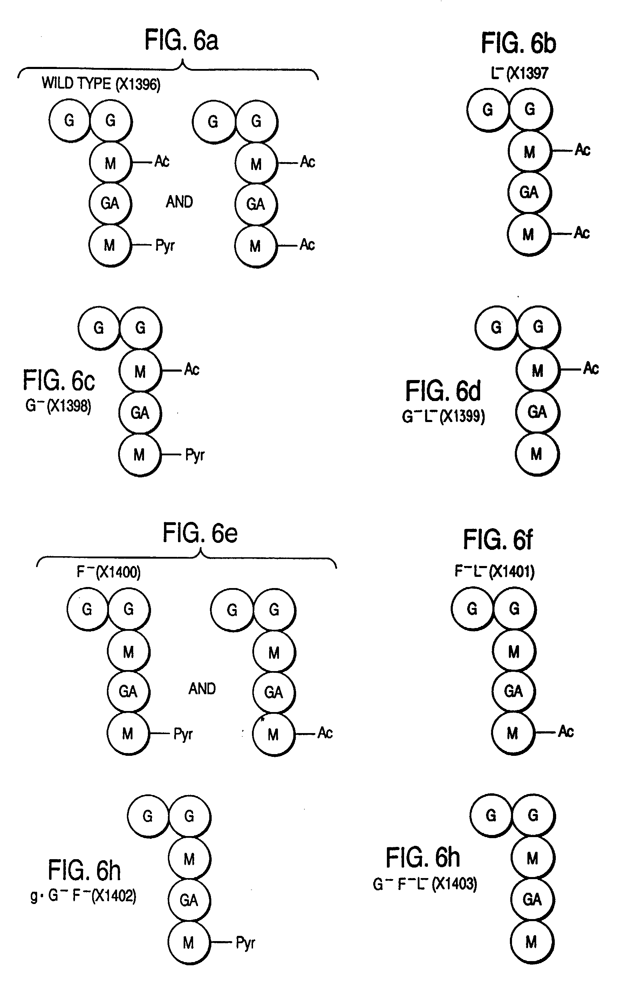

This example describes the repeating units that comprise the polysaccharide family that can be produced by genetic control of acetylation and pyruvylation of the pentasaccharide repeating unit of xanthan gum. The structures of these repeating units are shown in schematic form in Figure 6.
(a) Wild-type (X1396); Acetylase I⁺, Acetylase II⁺, Ketalase ⁺.
   Normal xanthan is extensively acetylated at the inner mannose residue and is frequently pyruvylated on the outer mannose residue. Contrary to general belief, a significant percentage (10-20) of the outer mannose residues of normal xanthan are acetylated. Thus, normal xanthan repeating units are heterogeneous with respect to modifications of the outer mannose, containing either pyruvate or acetate.
(b) L⁻(X1397); Acetylase I⁺, Acetylase II⁺, Ketalase⁻.
   This polymer contains no pyruvate and as a result is extensively acetylated at the outer mannose residue. The inner mannose residue is highly acetylated as in wild type.
(c) G⁻ (X1398); Acetylase I⁺, Acetylase II⁻, Ketalase⁺.
   This polymer is heavily acetylated on the inner mannose as in wild type, and the outer mannose is pyruvylated in the wild-type fashion. However, there is no acetylation of the outer mannose.
(d) G⁻, L⁻ (X1399); Acetylase I⁺, Acetylase II⁻, Ketalase⁻.
   This polymer has the high level wild-type acetylation of the inner mannose, but the outer mannose is unmodified.
(e) F⁻ (X1400); Acetylase I⁻, Acetylase II⁺, Ketalase⁺.
   The inner mannose of this polymer is unmodified, while the outer mannose is modified as in wild-type. That is, the outer mannose in generally pyruvylated, but a significant fraction of the outer mannose residues are acetylated instead.
(f) F⁻, L⁻ (X1401); Acetylase I⁻, Acetylase II⁺, Ketalase⁻.
   This polymer contains an unmodified inner mannose. The outer mannose is not pyruvylated but is heavily acetylated.
(g) F⁻, G⁻ (X1402); Acetylase I⁻, Acetylase II⁻, Ketalase⁺.
   This polymer is not acetylated at either the inner or outer mannose residues. Pyruvylation of the outer mannose occurs normally as in wild-type.
(h) F⁻, G⁻, L⁻ (X1403); Acetylase I⁻, Acetylase II⁻, Ketalase⁻.
   This polymer contains no acetate or pyruvate. Neither the inner nor the outer mannose residues are modified.

It will be apparent to those skilled in the art that various modifications and variations can be made in the processes and products of the present invention. Thus, it is intended that the present invention cover the modifications and variations of this invention provided they come within the scope of the appended claims .

## Claims (Claims for the following Contracting State(s): AT, BE, CH, LI, DE, DK, FR, GB, GR, IT, LU, NL, SE)

1. A process for preparing a water-soluble polysaccharide polymer comprising repeating pentamer units having a D-glucose: D-mannose: D-glucuronic acid ration of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, said process comprising:
(a) obtaining a Xanthomonas which is incapable of expressing a functional gene F protein of the xanthan gum gene cluster;
(b) culturing said Xanthomonas under conditions sufficient to produce said polysaccharide polymer.

2. The process of claim 1, wherein a portion of said outer mannose moieties are pyruvylated.

3. The process of claim 1, wherein said outer mannose moieties are not pyruvylated.

4. A process for preparing a water-soluble polysaccharide polymer comprising repeating pentamer units having a D-glucose: D-mannose: D-glucuronic acid ratio of about 2:2:1, wherein D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said outer mannose moieties are not acetylated and said inner mannose moieties are acetylated, said process comprising:
(a) obtaining a Xanthomonas which is incapable of expressing a functional gene G protein of the xanthan gum gene cluster;
(b) culturing said Xanthomonas under conditions sufficient to produce said polysaccharide polymer.

5. The process of claim 4, wherein a large portion of said inner mannose moieties are acetylated.

6. The process of any of claims 4 or 5, wherein a portion of said outer mannose moieties are pyruvylated.

7. The process of any of claims 4 or 5, wherein said outer mannose moieties are not pyruvylated.

8. The process of any of claims 1, 4 or 5, wherein said Xanthomonas is Xanthomonas campestris.

9. The process of claim 1, wherein said Xanthomonas is an acetylase I deficient mutant of Xanthomonas.

10. The process of claim 1, wherein said Xanthomonas is an acetylase I and ketalase deficient mutant of Xanthomonas.

11. The process of any of claims 4 or 5, wherein said Xanthomonas is an acetylase II deficient mutant of Xanthomonas.

12. The process of any of claims 4 or 5, wherein said Xanthomonas is an acetylase II and ketalase deficient mutant of Xanthomonas.

13. A water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated.

14. The polysaccharide polymer of claim 13, wherein a portion of said outer mannose moieties are pyruvylated.

15. The polysaccharide polymer of claim 13, wherein said outer mannose moieties are not pyruvylated.

16. A water-soluble polysaccharide polymer comprising repeating pentamer units having a D-glucose: D-mannose: D-glucuronic acid ratio of about 2:2:1, wherein D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein the outer mannose moieties are not acetylated and said inner mannose moieties are acetylated.

17. The polysaccharide polymer of claim 16, wherein a large portion of said inner mannose moieties are acetylated.

18. The polysaccharide polymer of any of claims 15 or 16, wherein said outer mannose moieties are not pyruvylated.

19. The polysaccharide polymer of any of claims 15 or 16, wherein a portion of said outer mannose moieties are pyruvylated.

20. A mutant Xanthomonas allowing production of the polysaccharide polymer of claim 13, wherein said Xanthomonas is an acetylase I deficient mutant of Xanthomonas.

21. A mutant Xanthomonas allowing production of the polysaccharide polymer of claim 13, wherein said Xanthomonas is an acetylase I and ketalase deficient mutant of Xanthomonas.

22. A mutant Xanthomonas allowing production of the polysaccharide polymer of claim 16, wherein said Xanthomonas is an acetylase II deficient mutant of Xanthomonas.

23. A mutant Xanthomonas allowing production of the polysaccharide polymer of claim 16, wherein said Xanthomonas is an acetylase II and ketalase deficient mutant of Xanthomonas.

## Claims (Claims for the following Contracting State(s): ES)

1. A process for preparing a water-soluble polysaccharide polymer comprising repeating pentamer units having a D-glucose: D-mannose: D-glucuronic acid ration of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, said process comprising:
(a) obtaining a Xanthomonas which is incapable of expressing a functional gene F protein of the xanthan gum gene cluster;
(b) culturing said Xanthomonas under conditions sufficient to produce said polysaccharide polymer.

2. The process of claim 1, wherein a portion of said outer mannose moieties are pyruvylated.

3. The process of claim 1, wherein said outer mannose moieties are not pyruvylated.

4. A process for preparing a water-soluble polysaccharide polymer comprising repeating pentamer units having a D-glucose: D-mannose: D-glucuronic acid ratio of about 2:2:1, wherein D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said outer mannose moieties are not acetylated and said inner mannose moieties are acetylated, said process comprising:
(a) obtaining a Xanthomonas which is incapable of expressing a functional gene G protein of the xanthan gum gene cluster;
(b) culturing said Xanthomonas under conditions sufficient to produce said polysaccharide polymer.

5. The process of claim 4, wherein a large portion of said inner mannose moieties are acetylated.

6. The process of any of claims 4 or 5, wherein a portion of said outer mannose moieties are pyruvylated.

7. The process of any of claims 4 or 5, wherein said outer mannose moieties are not pyruvylated.

8. The process of any of claims 1, 4 or 5, wherein said Xanthomonas is Xanthomonas campestris.

9. The process of claim 1, wherein said Xanthomonas is an acetylase I deficient mutant of Xanthomonas.

10. The process of claim 1, wherein said Xanthomonas is an acetylase I and ketalase deficient mutant of Xanthomonas.

11. The process of any of claims 4 or 5, wherein said Xanthomonas is an acetylase II deficient mutant of Xanthomonas.

12. The process of any of claims 4 or 5, wherein said Xanthomonas is an acetylase II and ketalase deficient mutant of Xanthomonas.

13. A mutant Xanthomonas allowing production of a water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, wherein said Xanthomonas is an acetylase I deficient mutant of Xanthomonas.

14. A mutant Xanthomonas according to claim 13 wherein said outer mannose moieties are not pyruvylated, wherein said Xanthomonas is an acetylase I and ketalase deficient mutant of Xanthomonas.

15. A mutant Xanthomonas allowing production of a water-soluble polysaccharide polymer comprising repeating pentamer units having a D-glucose: D-mannose: D-glucuronic acid ratio of about 2:2:1, wherein D-glucose moieties are linked in a beta[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein the outer mannose moieties are not acetylated and said inner mannose moieties are acetylated, wherein said Xanthomonas is an acetylase II deficient mutant of Xanthomonas.

16. A mutant Xanthomonas according to claim 15 wherein said outer mannose moieties are not pyruvylated, wherein said Xanthomonas is an acetylase II and ketalase deficient mutant of Xanthomonas.

17. A process for preparing a mutant Xanthomonas allowing production of a water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha-[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, wherein the gene coding for acetylase I is inactivated, resulting in an acetylase I deficient mutant of Xanthomonas.

18. A process for preparing a Xanthomonas allowing production of a water-soluble polysaccharide polymer comprising repeating pentamer units having a D-glucose: D-mannose: D-glucuronic acid ratio of about 2:2:1, wherein the D-glucose moieties are linked in a beta-[1,4] configuration, inner D-mannose moieties are linked in an alpha[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein said inner mannose moieties are not acetylated and a portion of said outer mannose moieties are acetylated, and wherein said outer mannose moieties are not pyruvylated, characterised in that the genes coding for acetylase I and ketalase are inactivated, resulting in an acetylase I and ketalase deficient mutant of Xanthomonas.

19. A process for preparing a Xanthomonas allowing production of a water-soluble polysaccharide polymer comprising repeating pentamer units having a D-glucose: D-mannose: D-glucuronic acid ratio of about 2:2:1, wherein D-glucose moieties are linked in a beta-[1,4] configuration, inner D-mannose moieties are linked in an alpha[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein the outer mannose moieties are not acetylated, and said inner mannose moieties are acetylated wherein the gene coding for acetylase II is inactivated, resulting in an acetylase II deficient mutant of Xanthomonas.

20. A process for preparing a Xanthomonas allowing production of a water-soluble polysaccharide polymer comprising repeating pentamer units having a D-glucose: D-mannose: D-glucuronic acid ratio of about 2:2:1, wherein D-glucose moieties are linked in a beta-[1,4] configuration, inner D-mannose moieties are linked in an alpha[1,3] configuration primarily to alternate glucose moieties, the D-glucuronic acid moieties are linked in a beta-[1,2] configuration to said inner mannose moieties, and outer mannose moieties are linked to said glucuronic acid moieties in a beta-[1,4] configuration, wherein the outer mannose moieties are not acetylated and said inner mannose moieties are acetylated, and wherein said outer mannose moieties are not pyruvylated, wherein the genes coding for acetylase II and ketalase are inactivated, resulting in an acetylase II and ketalase deficient mutant of Xanthomonas.

## Patentansprüche (Patentansprüche für folgende(n) Vertragsstaat(en): AT, BE, CH, LI, DE, DK, FR, GB, GR, IT, LU, NL, SE)

1. Verfahren zur Herstellung eines wasserlöslichen Polysaccharidpolymers mit sich wiederholenden Pentamereinheiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind, wobei das Verfahren umfaßt:
(a) Erhalten eines Xanthomonas, der nicht in der Lage ist, ein funktionelles Protein des F-Gens des Xanthangummi-Genclusters zu exprimieren;
(b) Kultivieren des Xanthomonas unter ausreichenden Bedingungen, um das Polysaccharidpolymer herzustellen.

2. Verfahren nach Anspruch 1, wobei ein Teil der äußeren Mannose-Komponenten pyruvyliert sind.

3. Verfahren nach Anspruch 1, wobei die äußeren Mannose-Komponenten nicht pyruvyliert sind.

4. Verfahren zur Herstellung eines wasserlöslichen Polysaccharidpolymers mit sich wiederholenden Pentamereinheiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind, wobei das Verfahren umfaßt:
(a) Erhalten eines Xanthomonas, der nicht in der Lage ist, ein funktionelles Protein des G-Gens des Xanthangummi-Genclusters zu exprimieren;
(b) Kultivieren des Xanthomonas unter ausreichenden Bedingungen, um das Polysaccharidpolymer herzustellen.

5. Verfahren nach Anspruch 4, wobei ein großer Teil der inneren Mannose-Komponenten acetyliert sind.

6. Verfahren nach Anspruch 4 oder 5, wobei ein Teil der äußeren Mannose-Komponenten pyruvyliert sind.

7. Verfahren nach Anspruch 4 oder 5, wobei die äußeren Mannose-Komponenten nicht pyruvyliert sind.

8. Verfahren nach einem der Ansprüche 1, 4 oder 5, wobei der Xanthomonas Xanthomonas campestris ist.

9. Verfahren nach Anspruch 1, wobei der Xanthomonas eine Acetylase I defiziente Mutante von Xanthomonas ist.

10. Verfahren nach Anspruch 1, wobei der Xanthomonas eine Acetylase I und Ketalase defiziente Mutante von Xanthomonas ist.

11. Verfahren nach Anspruch 4 oder 5, wobei der Xanthomonas eine Acetylase II defiziente Mutante von Xanthomonas ist.

12. Verfahren nach Anspruch 4 oder 5, wobei der Xanthomonas eine Acetylase II und Ketalase defiziente Mutante von Xanthomonas ist.

13. Wasserlösliches Polysaccharidpolymer mit sich wiederholenden Pentamerein-heiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind.

14. Polysaccharidpolymer nach Anspruch 13, wobei ein Teil der äußeren Mannose-Komponenten pyruvyliert sind.

15. Polysaccharidpolymer nach Anspruch 13, wobei die äußeren Mannose-Komponenten nicht pyruvyliert sind.

16. Wasserlösliches Polysaccharidpolymer mit sich wiederholenden Pentamereinheiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind.

17. Polysaccharidpolymer nach Anspruch 16, wobei ein großer Teil der Inneren Mannose-Komponenten acetyliert sind.

18. Polysaccharidpolymer nach Anspruch 15 oder 16, wobei die äußeren Mannose-Komponenten nicht pyruvyliert sind.

19. Polysaccharidpolymer nach Anspruch 15 oder 16, wobei ein Teil der äußeren Mannose-Komponenten pyruvyliert sind.

20. Xanthomonas-Mutante, die eine Herstellung des Polysaccharidpolymers nach Anspruch 13 erlaubt, wobei dieses Xanthomonas eine Acetylase I defiziente Mutante von Xanthomonas ist.

21. Xanthomonas-Mutante, die eine Herstellung des Polysaccharidpolymers nach Anspruch 13 erlaubt, wobei dieses Xanthomonas eine Acetylase I und Ketalase defiziente Mutante von Xanthomonas ist.

22. Xanthomonas-Mutante, die eine Herstellung des Polysaccharidpolymers nach Anspruch 16 erlaubt, wobei dieses Xanthomonas eine Acetylase II defiziente Mutante von Xanthomonas ist.

23. Xanthomonas-Mutante, die eine Herstellung des Polysaccharidpolymers nach Anspruch 16 erlaubt, wobei dieses Xanthomonas eine Acetylase II und Ketalase defiziente Mutante von Xanthomonas ist.

## Patentansprüche (Patentansprüche für folgende(n) Vertragsstaat(en): ES)

1. Verfahren zur Herstellung eines wasserlöslichen Polysaccharidpolymers mit sich wiederholenden Pentamereinheiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind, wobei das Verfahren umfaßt:
(a) Erhalten eines Xanthomonas, der nicht in der Lage ist, ein funktionelles Protein des F-Gens des Xanthangummi-Genclusters zu exprimieren;
(b) Kultivieren des Xanthomonas unter ausreichenden Bedingungen, um das Polysaccharidpolymer herzustellen.

2. Verfahren nach Anspruch 1, wobei ein Teil der äußeren Mannose-Komponenten pyruvyliert sind.

3. Verfahren nach Anspruch 1, wobei die äußeren Mannose-Komponenten nicht pyruvyliert sind.

4. Verfahren zur Herstellung eines wasserlöslichen Polysaccharidpolymers mit sich wiederholenden Pentamereinheiten, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind, wobei das Verfahren umfaßt:
(a) Erhalten eines Xanthomonas, der nicht in der Lage ist, ein funktionelles Protein des G-Gens des Xanthangummi-Genclusters zu exprimieren;
(b) Kultivieren des Xanthomonas unter ausreichenden Bedingungen, um das Polysaccharidpolymer herzustellen.

5. Verfahren nach Anspruch 4, wobei ein großer Teil der inneren Mannose-Komponenten acetyliert sind.

6. Verfahren nach Anspruch 4 oder 5, wobei ein Teil der äußeren Mannose-Komponenten pyruvyliert sind.

7. Verfahren nach Anspruch 4 oder 5, wobei die äußeren Mannose-Komponenten nicht pyruvyliert sind.

8. Verfahren nach einem der Ansprüche 1, 4 oder 5, wobei der Xanthomonas Xanthomonas campestris ist.

9. Verfahren nach Anspruch 1, wobei der Xanthomonas eine Acetylase I defiziente Mutante von Xanthomonas ist.

10. Verfahren nach Anspruch 1, wobei der Xanthomonas eine Acetylase I und Ketalase defiziente Mutante von Xanthomonas ist.

11. Verfahren nach Anspruch 4 oder 5, wobei der Xanthomonas eine Acetylase II defiziente Mutante von Xanthomonas ist.

12. Verfahren nach Anspruch 4 oder 5, wobei der Xanthomonas eine Acetylase II und Ketalase defiziente Mutante von Xanthomonas ist.

13. Xanthomonas-Mutante, welche die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind, und wobei dieses Xanthomonas eine Acetylase I defiziente Mutante von Xanthomonas ist.

14. Xanthomonas-Mutante nach Anspruch 13, wobei die äußeren Mannose-Komponenten nicht pyruvyliert sind, und wobei dieses Xanthomonas eine Acetylase I und Ketalase defiziente Mutante von Xanthomonas ist.

15. Xanthomonas-Mutante, welche die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind, und wobei dieses Xanthomonas eine Acetylase II defiziente Mutante von Xanthomonas ist.

16. Xanthomonas-Mutante nach Anspruch 15, wobei die äußeren Mannose-Komponenten nicht pyruvyliert sind, und wobei dieses Xanthomonas eine Acetylase II und Ketalase defiziente Mutante von Xanthomonas ist.

17. Verfahren zur Herstellung einer Xanthomonas-Mutante, welche die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind, und wobei das für die Acetylase I codierende Gen inaktiviert wird, was zu einer Acetylase I defizienten Mutante von Xanthomonas führt.

18. Verfahren zur Herstellung eines Xanthomonas, welches die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die inneren Mannose-Komponenten nicht acetyliert und ein Teil der äußeren Mannose-Komponenten acetyliert sind, und wobei die äußeren Mannose-Komponenten nicht pyruvyliert sind, dadurch gekennzeichnet, daß die für die Acetylase I und Ketalase codierenden Gene inaktiviert werden, was zu einer Acetylase I und Ketalase defizienten Mutante von Xanthomonas führt.

19. Verfahren zur Herstellung eines Xanthomonas, welches die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit alternierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind, und wobei das für die Acetylase II codierende Gen inaktiviert wird, was zu einer Acetylase II defizienten Mutante von Xanthomonas führt.

20. Verfahren zur Herstellung eines Xanthomonas, welches die Herstellung eines wasserlöslichen Polysaccharidpolymers mit sich wiederholenden Pentamereinheiten erlaubt, die ein D-Glucose:D-Mannose:D-Glucuronsäure - Verhältnis von etwa 2:2:1 haben, worin die D-Glucose-Komponenten in einer beta-[1,4]-Konfiguration verbunden sind, die inneren D-Mannose-Komponenten in einer alpha-[1,3]-Konfiguration primär mit altemierenden Glucose-Komponenten verbunden sind, die D-Glucuronsäure-Komponenten in einer beta-[1,2]-Konfiguration mit den inneren Mannose-Komponenten verbunden sind, und die äußeren Mannose-Komponenten in einer beta-[1,4]-Konfiguration mit den Glucuronsäure-Komponenten verbunden sind, wobei die äußeren Mannose-Komponenten nicht acetyliert und die inneren Mannose-Komponenten acetyliert sind, die äußeren Mannose-Komponenten nicht pyruvyliert sind, und wobei die für die Acetylase II und Ketalase codierenden Gene inaktiviert werden, was zu einer Acetylase II und Ketalase defizienten Mutante von Xanthomonas führt.

## Revendications (Revendications pour l'(les) Etat(s) contractant(s) suivant(s): AT, BE, CH, LI, DE, DK, FR, GB, GR, IT, LU, NL, SE)

1. Procédé de préparation d'un polymère polysaccharide soluble dans l'eau comprenant des unités pentamères répétitives présentant un rapport D-glucose: D-mannose : acide D-glucuronique d'environ 2:2:1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées, ledit procédé comprenant :
(a) l'obtention d'une Xanthomonas qui est incapable d'exprimer une protéine fonctionnelle de gène F du groupe de gènes de la gomme de xanthane ;
(b) la culture de ladite Xanthomonas dans des conditions suffisantes pour produire ledit polymère polysaccharide.

2. Procédé selon la revendication 1, dans lequel une partie desdites fractions de mannose extérieures sont pyruvylées.

3. Procédé selon la revendication 1, dans lequel lesdites fractions de mannose extérieures ne sont pas pyruvylées.

4. Procédé de préparation d'un polymère polysaccharide soluble dans l'eau comprenant des unités pentamères répétitives présentant un rapport D-glucose: D-mannose : acide D-glucuronique d'environ 2:2:1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose extérieures ne sont pas acétylées et lesdites fractions de mannose intérieures sont acétylées, ledit procédé comprenant :
(a) l'obtention d'une Xanthomonas qui est incapable d'exprimer une protéine fonctionnelle de gène G du groupe de gènes de la gomme de xanthane ;
(b) la culture de ladite Xanthomonas dans des conditions suffisantes pour produire ledit polymère polysaccharide.

5. Procédé selon la revendication 4, dans lequel une partie grande desdites fractions de mannose intérieures sont acétylées.

6. Procédé selon l'une quelconque des revendications 4 ou 5, dans lequel une partie desdites fractions de mannose extérieures sont pyruvylées.

7. Procédé selon l'une quelconque des revendications 4 ou 5, dans lequel lesdites fractions de mannose extérieures ne sont pas pyruvylées.

8. Procédé selon l'une quelconque des revendications 1, 4 ou 5, dans lequel ladite Xanthomonas est Xanthomonas campestris.

9. Procédé selon la revendication 1, dans lequel ladite Xanthomonas est un mutant de Xanthomonas déficient en acétylase I.

10. Procédé selon la revendication 1, dans lequel ladite Xanthomonas est un mutant de Xanthomonas déficient en acétylase I et en cétalase.

11. Procédé selon l'une quelconque des revendications 4 ou 5, dans lequel ladite Xanthomonas est un mutant de Xanthomonas déficient en acétylase Il.

12. Procédé selon l'une quelconque des revendications 4 ou 5, dans lequel ladite Xanthomonas est un mutant de Xanthomonas déficient en acétylase Il et en cétalase.

13. Polymère polysaccharide soluble dans l'eau comprenant des unités pentamères répétitives présentant un rapport D-glucose: D-mannose : acide D-glucuronique d'environ 2:2:1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées.

14. Polymère polysaccharide selon la revendication 13, dans lequel une partie desdites fractions de mannose extérieures sont pyruvylées.

15. Polymère polysaccharide selon la revendication 13, dans lequel lesdites fractions de mannose extérieures ne sont pas pyruvylées.

16. Polymère polysaccharide soluble dans l'eau comprenant des unités pentamères répétitives présentant un rapport D-glucose: D-mannose : acide D-glucuronique d'environ 2:2:1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose extérieures ne sont pas acétylées et lesdites fractions de mannose intérieures sont acétylées.

17. Polymère polysaccharide selon la revendication 16, dans lequel une partie grande desdites fractions de mannose intérieures sont acétylées.

18. Polymère polysaccharide selon l'une quelconque des revendications 15 ou 16, dans lequel lesdites fractions de mannose extérieures ne sont pas pyruvylées.

19. Polymère polysaccharide selon l'une quelconque des revendications 15 ou 16, dans lequel une partie desdites fractions de mannose extérieures sont pyruvylées.

20. Mutant de Xanthomonas permettant la production du polymère polysaccharide selon la revendication 13, ladite Xanthomonas étant un mutant de Xanthomonas déficient en acétylase I.

21. Mutant de Xanthomonas permettant la production du polymère polysaccharide selon la revendication 13, ladite Xanthomonas étant un mutant de Xanthomonas déficient en acétylase I et en cétalase.

22. Mutant de Xanthomonas permettant la production du polymère polysaccharide selon la revendication 16, ladite Xanthomonas étant un mutant de Xanthomonas déficient en acétylase II.

23. Mutant de Xanthomonas permettant la production du polymère polysaccharide selon la revendication 16, ladite Xanthomonas étant un mutant de Xanthomonas déficient en acétylase II et en cétalase.

## Revendications (Revendications pour l'(les) Etat(s) contractant(s) suivant(s): ES)

1. Procédé de préparation d'un polymère polysaccharide soluble dans l'eau comprenant des unités pentamères répétitives présentant un rapport D-glucose: D-mannose : acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées, ledit procédé comprenant :
(a) l'obtention d'une Xanthomonas qui est incapable d'exprimer une protéine fonctionnelle de gène F du groupe de gènes de la gomme de xanthane ;
(b) la culture de ladite Xanthomonas dans des conditions suffisantes pour produire ledit polymère polysaccharide.

2. Procédé selon la revendication 1, dans lequel une partie desdites fractions de mannose extérieures sont pyruvylées.

3. Procédé selon la revendication 1, dans lequel lesdites fractions de mannose extérieures ne sont pas pyruvylées.

4. Procédé de préparation d'un polymère polysaccharide soluble dans l'eau comprenant des unités pentamères répétitives présentant un rapport D-glucose: D-mannose : acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose extérieures ne sont pas acétylées et lesdites fractions de mannose intérieures sont acétylées, ledit procédé comprenant :
(a) l'obtention d'une Xanthomonas qui est incapable d'exprimer une protéine fonctionnelle de gène G du groupe de gènes de la gomme de xanthane ;
(b) la culture de ladite Xanthomonas dans des conditions suffisantes pour produire ledit polymère polysaccharide.

5. Procédé selon la revendication 4, dans lequel une partie grande desdites fractions de mannose intérieures sont acétylées.

6. Procédé selon l'une quelconque des revendications 4 ou 5, dans lequel une partie desdites fractions de mannose extérieures sont pyruvylées.

7. Procédé selon l'une quelconque des revendications 4 ou 5, dans lequel lesdites fractions de mannose extérieures ne sont pas pyruvylées.

8. Procédé selon l'une quelconque des revendications 1, 4 ou 5, dans lequel ladite Xanthomonas est Xanthomonas campestris.

9. Procédé selon la revendication 1, dans lequel ladite Xanthomonas est un mutant de Xanthomonas déficient en acétylase I.

10. Procédé selon la revendication 1, dans lequel ladite Xanthomonas est un mutant de Xanthomonas déficient en acétylase I et en cétalase.

11. Procédé selon l'une quelconque des revendications 4 ou 5, dans lequel ladite Xanthomonas est un mutant de Xanthomonas déficient en acétylase Il.

12. Procédé selon l'une quelconque des revendications 4 ou 5, dans lequel ladite Xanthomonas est un mutant de Xanthomonas déficient en acétylase II et en cétalase.

13. Mutant de Xanthomonas permettant la production d'un polymère polysaccharide soluble dans l'eau comprenant des unités pentamères répétitives présentant un rapport D-glucose: D-mannose:acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées, ladite Xanthomonas étant un mutant de Xanthomonas déficient en acétylase I.

14. Mutant de Xanthomonas selon la revendication 13, dans lequel lesdites fractions de mannose extérieures ne sont pas pyruvylées, ladite Xanthomonas étant un mutant de Xanthomonas déficient en acétylase I et en cétalase.

15. Mutant de Xanthomonas permettant la production d'un polymère polysaccharide soluble dans l'eau comprenant des unités pentamères répétitives présentant un rapport D-glucose: D-mannose: acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose extérieures ne sont pas acétylées et lesdites fractions de mannose intérieures sont acétylées, ladite Xanthomonas étant un mutant de Xanthomonas déficient en acétylase Il.

16. Mutant de Xanthomonas selon la revendication 15, dans lequel lesdites fractions de mannose extérieures ne sont pas pyruvylées, ladite Xanthomonas étant un mutant de Xanthomonas déficient en acétylase Il et en cétalase.

17. Procédé de préparation d'un mutant de Xanthomonas permettant la production d'un polymère polysaccharide soluble dans l'eau comprenant des unités pentamères répétitives présentant un rapport D-glucose: D-mannose : acide D-glucuronique d'environ 2:2:1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées, dans lequel le gène codant pour l'acétylase I est inactivé, entraînant un mutant de Xanthomonas déficient en acétylase I.

18. Procédé de préparation d'une Xanthomonas permettant la production d'un polymère polysaccharide soluble dans l'eau comprenant des unités pentamères répétitives présentant un rapport D-glucose: D-mannose : acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel lesdites fractions de mannose intérieures ne sont pas acétylées et une partie desdites fractions de mannose extérieures sont acétylées et dans lequel lesdites fractions de mannose extérieures ne sont pas pyruvylées, caractérisé en ce que les gènes codant pour l'acétylase I et la cétalase sont inactivés, entraînant un mutant de Xanthomonas déficient en acétylase I et en cétalase.

19. Procédé de préparation d'une Xanthomonas permettant la production d'un polymère polysaccharide soluble dans l'eau comprenant des unités pentamères répétitives présentant un rapport D-glucose: D-mannose : acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel les fractions de mannose extérieures ne sont pas acétylées et lesdites fractions de mannose intérieures sont acétylées, dans lequel le gène codant pour l'acétylase Il est inactivé, entraînant un mutant de Xanthomonas déficient en acétylase Il.

20. Procédé de préparation d'une Xanthomonas permettant la production d'un polymère polysaccharide soluble dans l'eau comprenant des unités pentamères répétitives présentant un rapport D-glucose: D-mannose : acide D-glucuronique d'environ 2 : 2 : 1, dans lequel les fractions de D-glucose sont liées en une configuration bêta-[1,4], les fractions de D-mannose intérieures sont liées en une configuration alpha-[1,3] principalement à des fractions de glucose alternantes, les fractions d'acide D-glucuronique sont liées en une configuration bêta-[1,2] auxdites fractions de mannose intérieures et les fractions de mannose extérieures sont liées auxdites fractions d'acide glucuronique en une configuration bêta-[1,4], dans lequel les fractions de mannose extérieures ne sont pas acétylées et lesdites fractions de mannose intérieures sont acétylées et dans lequel lesdites fractions de mannose extérieures ne sont pas pyruvylées, dans lequel les gènes codant pour l'acétylase Il et la cétalase sont inactivés, entraînant un mutant de Xanthomonas déficient en acétylase Il et en cétalase.
